Europäisches Patentamt

European Patent Office

Office européen des brevets

(19)

(11) Veröffentlichungsnummer: **0 047 485**
**A2**

(12) # EUROPÄISCHE PATENTANMELDUNG

(21) Anmeldenummer: 81106855.0

(22) Anmeldetag: 02.09.81

(51) Int. Cl.³: **C 12 N 1/10,** A 61 K 39/002, C 12 Q 1/00

(30) Priorität: 09.09.80 DE 3033776

(43) Veröffentlichungstag der Anmeldung: 17.03.82 Patentblatt 82/11

(84) Benannte Vertragsstaaten: **AT CH DE FR GB LI NL SE**

(71) Anmelder: **BEHRINGWERKE Aktiengesellschaft, Postfach 1140, D-3550 Marburg 1 (DE)**

(72) Erfinder: **Enders, Burkhard, Dr., Oberer Eichweg 12, D-3550 Marburg 1 (DE)**
Erfinder: **Hahn, Hans Heinrich, Waldweg 7, D-3550 Marburg 2 (DE)**
Erfinder: **Neunziger, Günter, Pappelweg 2, D-3550 Marburg 2 (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(54) Verfahren zur Vermehrung von Protozoen und ihre Verwendung.

(57) Es wird ein Verfahren zur Vermehrung von Protozoen beschrieben, in welchem mittels Dialyse Nährstoffe zugeführt und Stoffwechselprodukte entfernt werden. Die so gewonnenen Protozoen können in einem Impfstoff oder Diagnostikum verwendet werden.

EP 0 047 485 A2

- 1 -

BEHRINGWERKE AKTIENGESELLSCHAFT    Hoe 80/B 011 Dr.HA/B1


Verfahren zur Vermehrung von Protozoen und ihre Verwendung


Die Erfindung betrifft ein Verfahren zur Vermehrung von Protozoen, besonders von Plasmodien, insbesondere Plasmodium falciparum, in einem Serum und gegebenenfalls Wirtszellen enthaltenden Medium, welchem mittels Dialyse Nährstoffe zugeführt und aus welchem gleichzeitig Stoffwechselprodukte entfernt werden.

Es sind Verfahren zur Vermehrung von Plasmodien in Erythrozyten bekannt, in welchen das vollständige serumhaltige Nährmedium kontinuierlich oder automatisch periodisch erneuert wird. So wird in dem von TRAGER, W., J.Protozool. 26 (1), 1979, S. 125-129, beschriebenen Verfahren das Kulturmedium kontinuierlich über die Suspension von Plasmodien und Erythrozyten geführt. Das von JENSEN, J.B., TRAGER, W. and DOHERTY, J. in Experimental Parasitology 48, 1979, S. 36-41 beschriebene Verfahren arbeitet mit einer Vorrichtung, die es gestattet, das Kulturmedium automatisch periodisch von der Suspension von Plasmodien und Erythrozyten abzudekantieren und frisches Kulturmedium zuzuführen.

Diese Verfahren haben den Nachteil, daß beim Wechsel des mit inhibierenden Stoffwechselprodukten angereicherten Kulturmediums auch wertvolles noch nicht verbrauchtes Humanserum sowie Protozoen (freie Merozoiten) und Wirtszellen (Erythrozyten) verlorengehen. Außerdem sind diese Methoden nicht für eine Vermehrung in größerem Maßstab geeignet.

- 2 -

Es stellte sich daher die Aufgabe, ein Verfahren zu finden, mittels welchem Protozoen, wie beispielsweise für eine Impfstoffherstellung erforderlich, in größeren Mengen und hoher Ausbeute in wirtschaftlicher Weise vermehrt werden können.

Diese Aufgabe konnte dadurch gelöst werden, daß überraschend gefunden wurde, daß sich Protozoen, besonders Plasmodien, insbesondere Plasmodium falciparum, mit guter Ausbeute vermehren lassen, wenn man die Serum enthaltende Kultursuspension von Protozoen und gegebenenfalls Wirtszellen durch eine Dialyse-Membran trennt von einer serumfreien Lösung, welche die niedermolekularen zur Vermehrung nötigen Nährstoffe enthält. Diese Lösung kann gewechselt werden, ohne daß Protozoen, Wirtszellen oder Serum verlorengehen. Außerdem werden die in der Kultur angereicherten, das Wachstum der Protozoen hemmenden, niedermolekularen metabolischen Produkte, vornehmlich Lactat, durch das Dialyseverfahren entfernt.

Gegenstand der Anmeldung ist demnach ein Verfahren zur Vermehrung von Protozoen, besonders Plasmodien, insbesondere Plasmodium falciparum, dadurch gekennzeichnet, daß eine Suspension, die mindestens Protozoen und Serum sowie gegebenenfalls Wirtszellen enthält, durch eine lediglich für niedermolekulare Stoffe durchlässige Membran getrennt ist von einer Lösung, die niedermolekulare für die Vermehrung der Protozoen nötige Nährstoffe enthält und andererseits schädliche niedermolekulare Stoffwechselprodukte aufnehmen kann.

Plasmodium falciparum ist der Erreger der Malaria, und Verfahren zu seiner Kultivierung haben eine große Bedeutung für die Suche nach Mitteln zur Bekämpfung dieser verbreiteten Krankheit.

Es ist bekannt, daß zum Wachstum und zur Vermehrung von Plasmodien ein Kulturmedium erforderlich ist, das Humanserum und Erythrozyten der mit dem Serum kompatiblen Blutgruppe enthält, und das von Zeit zu Zeit erneuert werden muß. Es war jedoch nicht bekannt, daß nicht das komplette Kulturmedium ausgetauscht werden muß, sondern daß es überraschenderweise genügt, für eine Zu- und Abführung der niedermolekularen Anteile (essentielle Aminosäuren, inhibierende Stoffwechselprodukte) zu sorgen. Das erfindungsgemäße Verfahren besteht darin, daß eine Suspension von Protozoen und gegebenenfalls Wirtszellen in einem definierten Medium die für die Kultivierung nötigen hochmolekularen Stoffe (z.B. Serumproteine) sowie gegebenenfalls weitere Bestandteile enthält und durch eine Membran, die nur niedermolekulare Stoffe passieren läßt, getrennt ist von einer Lösung, welche nur niedermolekulare für die Kultivierung nötige Stoffe enthält.

Als Protozoen kommen in Frage: Plasmodien, Piroplasmen, Trypanosomen, Leishmanien, Trichomonaden, Amoeben, Toxoplasmen.

Besonders geeignet ist das Verfahren für die Plasmodien: P. falciparum, P. fragile, P. malariae, P. knowlesi, P. gallinaceum, insbesondere P. falciparum.

Die niedermolekularen Anteile von Serum und gegebenenfalls Wirtszellen enthaltenden Nährmedien, die für die Kultivierung von Protozoen geeignet sind, sind bekannt. Im wesentlichen sind dies Aminosäuren, Vitamine, Pepton und physiologisch verträgliche Salze. Für Plasmodium falciparum ist beispielsweise ein unter der Bezeichnung RPMI 1640 kommerziell erhältliches Medium, das nachstehend beschrieben ist, verwendbar. Weiterhin ist beispielsweise geeignet modifiziertes "Medium 199" (Haynes et al., 1976).

- 4 -

Bestandteile solcher niedermolekularen Anteile von Nährmedien, die für die Kultivierung von Protozoen verwendet
werden, sind beispielsweise :

1 1 RPMI 1640 enthaltend                     mg/l

| | |
|---|---|
| L-Arginin | 200,0 |
| L-Asparagin | 50,0 |
| L-Asparaginsäure | 20,0 |
| L-Cystin | 50,0 |
| L-Valin | 20,0 |
| L-Glutaminsäure | 20,0 |
| L-Glutamin | 300,0 |
| Glutathion (reduziert) | 1,0 |
| Glycin | 10,0 |
| L-Histidin | 15,0 |
| L-Hydroxyprolin | 20,0 |
| L-Isoleucin | 50,0 |
| L-Leucin | 50,0 |
| L-Lysin-HCl | 40,0 |
| L-Methionin | 15,0 |
| L-Phenylalanin | 15,0 |
| L-Prolin | 20,0 |
| L-Serin | 30,0 |
| L-Threonin | 20,0 |
| L-Tryptophan | 5,0 |
| L-Tyrosin | 20,0 |
| Folsäure | 1,0 |
| i-Inositol | 35,0 |
| Biotin | 0,2 |
| $B_{12}$ | 0,005 |
| Calcium-Pantothenat | 0,25 |
| Cholin-Chlorid | 3,0 |

|                              | mg/l   |
|------------------------------|--------|
| Nicotinamid                  | 1,0    |
| Para-Amino Benzolsäure       | 1,0    |
| Pyridoxin-HCl                | 1,0    |
| Riboflavin                   | 0,2    |
| Thiamin-HCl                  | 1,0    |
| Calcium-Nitrat.4H$_2$O       | 100,0  |
| Di-Natrium-Phosphat.7H$_2$O  | 1512,0 |
| Glucose                      | 2000,0 |
| Magnesium-Sulfat.7H$_2$O     | 100,0  |
| Phenolrot                    | 5,0    |
| Kalium-Chlorid               | 400,0  |
| Natrium-Bicarbonat           | 2000,0 |
| Natrium-Chlorid              | 6000,0 |

dazu  5,94 g  (25 mM) Hepes

42   ml 5%ige NaHCO$_3$-Lösung und

10   ml Na-Glutaminat-Lösung (200 mmol).

Als Serum wird bevorzugt eingesetzt:  Humanserum A$_1$, Rh$^-$;  Humanserum O.

Als Wirtszellen werden verwendet:

Erythrozyten:  für Plasmodien und Piroplasmen

Säuger- oder Hühnerfibroblasten: für Trypanosoma cruzi

Makrophagen:  für Leishmania spp.

Lymphoblasten:  für Theileria spp.

Insektenzellen: für Plasmodien

Karzinomzellen: für Toxoplasma grudii

Falls Erythrozyten verwendet werden, sind brauchbar:

Humanerythrozyten A, Rh$^-$;  Humanerythrozyten O .

Das folgende Beispiel soll die Erfindung erläutern:

In einem Fermenter wird eine sedimentierte Schicht gewaschener menschlicher Erythrozyten ($A_1$, $Rh^-$) mit einem definierten Medium (z.B. RPMI 1640) überschichtet. Die Menge des Mediums wird so berechnet, daß eine 1 - 10 %ige Erythrozytensuspension entsteht. Das Medium RPMI 1640 wird mit 5 - 10 % Humanserum der kompatiblen Gruppe substituiert. In den Fermenter wird eine Gasmischung bestehend aus 2 - 5 % $CO_2$, 5 - 10 % $O_2$ und 85 - 93 % $N_2$ zur Oberflächenbelüftung eingeleitet. In einem kontinuierlichen Durchfluß strömt das Medium über die ruhende Schicht der Erythrozyten, nachdem es eine Dialysekammer (z.B. Amicon Hollow Fiber) durchlaufen hat. Auf der Gegenseite der Membran wird in einem zweiten Kreislaufsystem die 5 - 10fache Menge RPMI 1640 ohne Serum, Erythrozyten und Protozoen in entgegengesetzter Richtung gepumpt. Durch die Dialysemembran erfolgt der Austausch essentieller, niedermolekularer Nährsubstanzen, die infolge des Wachstums der Plasmodien und im geringeren Maße auch durch die Wirtszellen verbraucht werden, aus dem Vorratsgefäß in die Fermenterkultur sowie der Entzug von niedermolekularen inhibierenden Stoffwechselprodukten (z.B. Lactat) in das Außendialysat.

Die unter anderem durch den Parasitenstoffwechsel angereicherte Milchsäure (Lactat), die normalerweise eine Wachstums-inhibierende lokale pH-Änderung im Medium bedingt, wird durch die Dialysemethode entfernt, wodurch günstigere Wachstumsbedingungen erreicht werden (pH-Stabilität, Erhöhung des angebotenen Erythrozytenanteils und dadurch höhere Ausbeute an Malariaparasiten, Einsparung an unverbrauchten hochmole-kularen Serumbestandteilen) :

| Methode | Erythro-zyten-Sediment | Initial-parasi-tämie | Vol. Medium | Vol. Serum | Endpara-sitämie | total Anzahl Parasiten |
|---------|------------------------|----------------------|-------------|------------|-----------------|------------------------|
| TRAGER/ JENSEN | 5 ml | 1 % | 300 ml | 30 ml | 10 % | $45 \times 10^9$ |
| Dialyse-Fermenter | 5 ml | 2 % | 250 ml | 25 ml | >50 % | $>12 \times 10^{10}$ |

Danach hergestellte Parasiten, vor allem Plasmodium falciparum, sind zur Herstellung eines antiparasitären (Malaria-) Impfstoffes geeignet. Die nach dem Wachstum geernteten Plasmodium falciparum Merozoiten (Plasmodien-Stadien in den Erythrozyten werden zum Beispiel durch Hämolyse aus der Wirtszelle befreit) werden ankonzentriert, attenuiert (chemisch oder physikalisch) und in eine Impfstoff gerechte Form gebracht.

Gegenstand der Erfindung ist demnach insbesondere die Verwendung der nach dem erfindungsgemäßen Verfahren hergestellten Parasiten als wesentlicher Wirkstoff in einem Impfstoff oder in einem Diagnostikum.

Patentansprüche :

1. Verfahren zur Vermehrung von Protozoen in einer Suspension, welche Protozoen, Serum und gegebenenfalls Wirtszellen enthält, dadurch gekennzeichnet, daß die Suspension, getrennt durch eine Membran, die für niedermolekulare Stoffe durchlässig ist, mit einer Lösung von niedermolekularen Stoffen in Verbindung steht, die für die Vermehrung nötig sind, und daß diese Lösung inhibierende Stoffwechselprodukte aus der Suspension aufnimmt.

2. Verwendung der nach dem Verfahren des Anspruchs 1 hergestellten Protozoen zur Herstellung eines Impfstoffes oder eines Diagnostikums.